# EUROPEAN PATENT APPLICATION

(11) **EP 4 635 505 A1**
(43) Date of publication of application: **22.10.2025**
(21) Application number: 23903412.7
(22) Date of filing: 07.12.2023
(51) Int. Cl.: A61K 35/33, A61K 31/155, A61K 31/4439, A61P 11/00

(54) **THERAPEUTIC AGENT FOR CHRONIC RESPIRATORY DISEASES**

(30) Priority: 16.12.2022 JP 2022201291
(71) Applicant: The Jikei University, Tokyo 105-8461 (JP)
(72) Inventor: FUJITA Yu, Tokyo 105-8461 (JP); FUJIMOTO Shota, Tokyo 105-8461 (JP); ARAYA Jun, Tokyo 105-8461 (JP)
(74) Representative: Cabinet Becker et Associés
(86) International application number: PCT/JP2023/043834
(87) International publication number: WO 2024/128125

(57) **Abstract**

A therapeutic agent for chronic respiratory diseases, containing extracellular vesicles derived from a lipofibroblast as an active component.

## Description

### TECHNICAL FIELD

The present invention relates to a therapeutic agent for chronic respiratory diseases.

### BACKGROUND ART

Chronic obstructive pulmonary disease (COPD) is a disease characterized by chronic inflammation and pulmonary emphysema due to environmental factors mainly including smoking exposure, and is a disease associated with pneumonia, acute exacerbation, lung cancer, and the like.

As a current drug therapy for COPD, a bronchodilator that dilates the bronchus and makes breathing easier is mainly used (see Patent Document 1).

Idiopathic pulmonary fibrosis (IPF) is a disease caused by excessive production of extracellular matrix by myofibroblasts induced by chronic inflammation or tissue damage, and the life prognosis is very poor with a 5-year survival rate of about 20% to 40%. As a current drug treatment for IPF, there is an antifibrotic agent that suppresses the progression of fibrosis, but there is still no fundamental treatment.

Bronchial asthma is a disease mainly characterized by airway inflammation, airway hyperresponsiveness, reversible airway narrowing, and airway remodeling. Chronic airway inflammation induces a change of architecture (remodeling) associated with airway fibrosis. The current treatment with inhaled steroids or bronchodilators is not a fundamental treatment method, and although the treatment of steroid-resistant bronchial asthma has progressed with biological products that have appeared recently, there is still no fundamental treatment that improves airway remodeling.

Cystic fibrosis (CF) is a genetic disease in which a cystic fibrosis transmembrane conductance regulator (CFTR) is the causative molecule, and secretory glands produce abnormal secretions, which damage the lungs and the digestive tract. Thereafter, the patient suffers from respiratory failure due to repeated respiratory infections. The basic treatment is to combine chest physical therapy (postural drainage, tapping), an expectorant, and a bronchodilator, and to use an appropriate antibiotic for respiratory infection, but there is no fundamental treatment.

### Citation List

### Patent Document

Patent Document 1: Published Japanese Translation No. 2022-522229 of the PCT International Publication

### SUMMARY OF INVENTION

### Technical Problem

However, the treatment of chronic respiratory diseases such as COPD, IPF, bronchial asthma, and CF is still difficult, and further improvement of treatment methods is desired.

Therefore, an object of the present invention is to provide a therapeutic agent for chronic respiratory diseases and a composition for treating chronic respiratory diseases, which have a new therapeutic effect.

### Solution to Problem

The present invention includes the following aspects.
[1] A therapeutic agent for chronic respiratory diseases, containing extracellular vesicles (EVs) from a lipofibroblast as an active component.
[2] The therapeutic agent for chronic respiratory diseases according to [1], in which the EVs from a lipofibroblast express L-type amino acid transporter 1 (LAT1).
[3] The therapeutic agent for chronic respiratory diseases according to [1], in which the lipofibroblast is differentiated from a lung fibroblast by a metabolic regulating agent.
[4] The therapeutic agent for chronic respiratory diseases according to [3], in which the metabolic regulating agent is at least one selected from the group consisting of Metformin, Rosiglitazone, Pioglitazone, Ciglitazone, Dibutyryl cyclic AMP (DBcAMP), Parathyroid hormone-related peptide (PTHrP), Pemafibrate, and Pravastatin.
[5] The therapeutic agent for chronic respiratory diseases according to [3], in which the metabolic regulating agent is Rosiglitazone.
[6] The therapeutic agent for chronic respiratory diseases according to [1], in which the therapeutic agent has an anti-fibrotic effect.
[7] The therapeutic agent for chronic respiratory diseases according to [1], in which the therapeutic agent has an anti-aging effect.
[8] The therapeutic agent for chronic respiratory diseases according to [1], in which the therapeutic agent has a proliferation effect on epithelial cells.
[9] A composition for treating chronic respiratory diseases, containing the therapeutic agent for chronic respiratory diseases according to any one of [1] to [8] and an additive that is pharmaceutically acceptable.
[10] A quality evaluation kit for the therapeutic agent for chronic respiratory diseases according to any one of [1] to [8], the quality evaluation kit containing an anti-LAT1 antibody.
[11] A quality evaluation method for the therapeutic agent for chronic respiratory diseases according to any one of [1] to [8], the quality evaluation method including using an anti-LAT1 antibody.

### Advantageous Effects of Invention

According to the present invention, the present invention contributes to the treatment of chronic respiratory diseases.

### BRIEF DESCRIPTION OF DRAWINGS

[FIG. 1] A Result of staining lipid droplets in the cytoplasm using LipidTOX (registered trademark).
[FIG. 2] (A) A result of measuring cells stained with LipidTOX using flow cytometry. (B) A quantitative result of flow cytometry.
[FIG. 3] A result of performing the same experiment as in FIG. 2 using primary culture cells of lung fibroblasts derived from a COPD patient and an idiopathic pulmonary fibrosis (IPF) patient.
[FIG. 4] A result of examining the expression of ADRP, CD36, and APOE in lung fibroblasts derived from a healthy subject, which has been cultured in the presence of a metabolic regulating agent.
[FIG. 5] A result of examining the expression of each protein in cells by Western blot after adding 2 ng/mL of TGFβ1 and 10 µg/mL of EVs to primary culture cells of lung fibroblasts derived from a healthy subject.
[FIG. 6] A result of quantifying the signal intensity of FIG. 5. LF-EVs represent lung fibroblast-derived EVs, LiF-EVs (M) represent Metformin-induced lipofibroblast-derived EVs, and LiF-EVs (R) represent Rosiglitazone-induced lipofibroblast-derived EVs.
[FIG. 7] A result of performing the same experiment as in FIG. 5 using each of the metabolic regulating agents.
[FIG. 8] A result of quantifying the signal intensity of FIG. 7.
[FIG. 9] A result of performing the same experiment as in FIG. 5 using an existing antifibrotic agent.
[FIG. 10] A result of quantifying the signal intensity of FIG. 9. LiF-EVs represent lipofibroblast-derived EVs, PFD represents Pirfenidon, and NTD represents Nintedanib.
[FIG. 11] (A) A schedule for the administration of lung fibroblast-derived EVs using a pulmonary fibrosis model. (B) A result of measuring the body weight of each administration group. BLM represents Bleomycin, LF-EVs represent lung fibroblast-derived EVs, and LiF-EVs represent lipofibroblast-derived EVs.
[FIG. 12] A result of HE staining and Masson trichrome staining performed on pulmonary tissue sections of a pulmonary fibrosis model mouse after the administration of lung fibroblast-derived EVs. HE represents Hematoxylin and Eosin.
[FIG. 13] (A) A quantitative result of FIG. 12. (B) A result of a hydroxyproline assay using a homogenized lung sample of a pulmonary fibrosis model mouse after the administration of lung fibroblast-derived EVs. BLM represents Bleomycin, LF-EVs represent lung fibroblast-derived EVs, and LiF-EVs represent lipofibroblast-derived EVs.
[FIG. 14] A result of confirming expression of each protein in cells by Western blot after culturing human bronchial epithelial cells for 48 hours with the addition of 1% of cigarette smoke extract (CSE) and 10 µg/mL of EVs.
[FIG. 15] (A) A quantitative result in which the expression level of p21 in FIG. 14 is corrected by the expression level of βactin. (B) A quantitative result in which the expression level of p16 in FIG. 14 is corrected by the expression level of βactin. CSE represents cigarette smoke extract, LF-EVs represent lung fibroblast-derived EVs, LiF-EVs (M) represent Metformin-induced lipofibroblast-derived EVs, and LiF-EVs (R) represent Rosiglitazone-induced lipofibroblast-derived EVs.
[FIG. 16] A result of performing SA-β-gal staining after culturing human bronchial epithelial cells for 48 hours with the addition of 1% of cigarette smoke extract (CSE) and 10 µg/mL of EVs.
[FIG. 17] A quantitative result of FIG. 16. CSE represents cigarette smoke extract, LF-EVs represent lung fibroblast-derived EVs, LiF-EVs (M) represent Metformin-induced lipofibroblast-derived EVs, and LiF-EVs (R) represent Rosiglitazone-induced lipofibroblast-derived EVs.
[FIG. 18] A diagram showing a schedule for administering lipofibroblast-derived EVs to smoking-exposed model mouse. it represents intratracheal administration, poly(I:C) represents polyinosinic-polycytidylic acid sodium salt, in represents intranasal administration, LF-EVs represent lung fibroblast-derived EVs, and LiF-EVs represent lipofibroblast-derived EVs.
[FIG. 19] (A) A graph showing a change in body weight of a smoking-exposed model mouse to which lipofibroblast-derived EVs have been administered. (B) A graph showing the body weight of the smoking-exposed model mouse on the 23rd day after the administration of the lipofibroblast-derived EVs. "it" represents intratracheal administration, poly IC represents polyinosinic-polycytidylic acid sodium salt, "in" represents intranasal administration, LF-EVs represent lung fibroblast-derived EVs, and LiF-EVs represent lipofibroblast-derived EVs.
[FIG. 20] A result of a respiratory function test performed using a flexiVent on a smoking-exposed model mouse to which lipofibroblast-derived EVs have been administered.
[FIG. 21] A result of HE staining and Masson trichrome staining performed on a pulmonary tissue section of a smoking-exposed model mouse to which lipofibroblast-derived EVs have been administered. "it" represents intratracheal administration, poly IC represents polyinosinic-polycytidylic acid sodium salt, "in" represents intranasal administration, LF-EVs represent lung fibroblast-derived EVs, LiF-EVs represent lipofibroblast-derived EVs, and HE represents Hematoxylin and Eosin.
[FIG. 22] A result of observing a wall thickness of a bronchus having a diameter of about 200 µm in the pulmonary tissue section of a smoking-exposed model mouse to which lipofibroblast-derived EVs have been administered.
[FIG. 23] (A) A result of quantifying the diameter of the wall thickness of the bronchus in FIG. 22. (B) A result of quantifying the wall thickness of the bronchus/the diameter of the small airway in FIG. 22. "it" represents intratracheal administration, poly IC represents polyinosinic-polycytidylic acid sodium salt, "in" represents intranasal administration, LF-EVs represent lung fibroblast-derived EVs, and LiF-EVs represent lipofibroblast-derived EVs.
[FIG. 24] (A) A microscopic observation image of human bronchial epithelial cells (HBEC) subjected to planar culture with the addition of lipofibroblast-derived EVs. (B) A result of the CCK-8 assay. LF-EVs represent lung fibroblast-derived EVs, and LiF-EVs represent lipofibroblast-derived EVs.
[FIG. 25] (A) A microscopic observation image of human small airway epithelial cells (HSAEC) subjected to planar culture with the addition of lipofibroblast-derived EVs. (B) A result of the CCK-8 assay. LF-EVs represent lung fibroblast-derived EVs, and LiF-EVs represent lipofibroblast-derived EVs.
[FIG. 26] (A) A microscopic observation image of HBEC subjected to 3D culture with the addition of lipofibroblast-derived EVs. (B) Results of the organoid size and the CCK-8 assay. LF-EVs represent lung fibroblast-derived EVs, and LiF-EVs represent lipofibroblast-derived EVs.
[FIG. 27] (A) A microscopic observation image of alveolar type II cells (AT2) subjected to 3D culture with the addition of lipofibroblast-derived EVs. (B) A result of the organoid size. LF-EVs represent lung fibroblast-derived EVs, and LiF-EVs represent lipofibroblast-derived EVs.
[FIG. 28] A diagram showing a schedule for administering lipofibroblast-derived EVs to 6-month smoking-exposed model mouse. The administration of the lipofibroblast-derived EVs is started 12 weeks after the smoking exposure. The administration of EVs is performed by intratracheal administration of 1 × 10⁹ particles/body of EVs once a week.
[FIG. 29] A result of a respiratory function test performed on a 6-month smoking-exposed model mouse.
[FIG. 30] (A) A Diff-Quik-stained image of inflammatory cells in a bronchoalveolar lavage (BAL) fluid. (B) A graph showing the total number of cells, the number of macrophages, the number of neutrophils, and the number of lymphocytes.
[FIG. 31] (A) An HE-stained image of a pulmonary tissue of a 6-month smoking mouse. (B) A graph showing a quantitative result of a mean linear intercept (MLI).
[FIG. 32] (A) A Masson trichrome-stained image of a pulmonary tissue of a 6-month smoking mouse. (B) A graph showing a quantitative result of a bronchial wall thickness.
[FIG. 33] A diagram showing a scheme for analyzing proteins in lipofibroblast-derived EVs.
[FIG. 34] A result of Western blot analysis performed on lung fibroblast-derived EVs and lipofibroblast-derived EVs.
[FIG. 35] (A) A result of confirming a change in the expression level of LAT1 with the addition of lipofibroblast-derived EVs to HBEC cells. (B) A result of confirming a change in the expression level of LAT1 with the addition of lipofibroblast-derived EVs to AT2 cells.
[FIG. 36] (A) A result of evaluating a change in the uptake of amino acids in cells by LAT1 in HBEC cells by an amino acid uptake assay. (B) A result of evaluating a change in the uptake of amino acids in cells by LAT1 in AT2 cells by an amino acid uptake assay.
[FIG. 37] A result of evaluating a change in the uptake of amino acids in cells by LAT1 in HBEC cells by a ³H-L-Leu uptake experiment.
[FIG. 38] (A) A result of examining the expression of each protein by Western blot after adding 1% of cigarette smoke extract (CSE), 10 µg/mL of EVs, and BCH to HBEC cells and culturing for 48 hours. (B) A result of the CCK-8 assay of AT2 cells subjected to 3D culture using Matrigel.

### DESCRIPTION OF EMBODIMENTS

### [Therapeutic agent for chronic respiratory diseases]

In one embodiment, the present invention provides a therapeutic agent for chronic respiratory diseases, containing extracellular vesicles (EVs) derived from a lipofibroblast as an active component. The lipofibroblast is a fibroblast that acts on the differentiation of AT2 and the control of homeostasis by a humoral factor. It is preferable that the lipofibroblast is differentiated from a lung fibroblast by a metabolic regulating agent.

Examples of the chronic respiratory diseases include chronic obstructive pulmonary disease (COPD), idiopathic pulmonary fibrosis (IPF), bronchial asthma, cystic fibrosis (CF), pulmonary hypertension, acute respiratory distress syndrome (ARDS), radiation pneumonitis, pulmonary sarcoidosis, interstitial pneumonia associated with connective tissue diseases (for example, systemic lupus erythematosus (SLE), vasculitis, scleroderma, Sjögren's syndrome, rheumatoid arthritis, and sarcoidosis), and the like.

Examples of the metabolic regulating agent include Metformin, Rosiglitazone, Pioglitazone, Ciglitazone, DBcAMP, PTHrP, Pemafibrate, and Pravastatin, and at least one selected from the group consisting of Metformin, Rosiglitazone, Pioglitazone, Ciglitazone, DBcAMP, PTHrP, Pemafibrate, and Pravastatin is preferable and Rosiglitazone is more preferable.

In the present embodiment, extracellular vesicles (hereinafter, also referred to as EVs) refer to vesicles secreted from cells. EVs also include vesicles such as exosomes, microvesicles, and apoptotic bodies. The surface of EVs contains lipids and proteins derived from cell membranes, and the inside contains intracellular substances such as nucleic acids and proteins.

It is preferable that the lipofibroblast-derived EVs express L-type amino acid transporter 1 (LAT1) (Gene ID: 8140). The Gene ID represents a registration number of a Gene database (URL: http://www.ncbi.nlm.nih.gov/gene/). LAT1 is a 12-transmembrane type membrane protein and is an amino acid transporter that transports large neutral amino acids such as leucine, isoleucine, and valine. As described later in Examples, LAT1 has been found as a protein having an expression difference of 1.5 times or more in EVs of a lipofibroblast as compared with EVs of a lung fibroblast. The present invention suggests that the LAT1 which is an amino acid transporter is delivered to cells by the administration of the lipofibroblast-derived EVs and functions on the recipient cells.

In the preparation of EVs, for example, lipofibroblasts or lung fibroblasts to which a metabolic regulating agent has been added can be cultured in a culture medium and the EVs can be recovered from the culture supernatant. The culture temperature is a culture temperature of mammalian cells, and is preferably 30°C to 40°C. The culture period is, for example, preferably 1 to 10 days and more preferably 1 to 5 days. The lung fibroblasts may be cultured in the presence of the metabolic regulating agent for preferably 1 to 5 days and more preferably 1 to 3 days, transformed into lipofibroblasts, and then further cultured for preferably 1 to 3 days and more preferably for 1 or 2 days.

Examples of the method of recovering EVs from the culture solution include an ultracentrifugation method, an immunoprecipitation method, a gel filtration method, an ultrafiltration method, a polymer precipitation method, an HPLC method, a FACS method, and the like, and an ultracentrifugation method is preferable. In the ultracentrifugation method, by combining rough centrifugation and filtration through a 0.22 µm filter, EVs mainly containing exosomes having a diameter of about 30 to 200 nm can be recovered.

In the present embodiment, the cell may be a primary cultured cell or a passaged cell. The biological species from which the cell is derived is not limited, and a mammal is preferable. Examples of the mammal include a human, a chimpanzee, a monkey, a rat, a mouse, a pig, a cow, a horse, a sheep, a goat, a dog, a cat, and the like, and a human or a mouse is preferable and a human is more preferable.

The amount of EVs contained in the therapeutic agent according to the present embodiment can be appropriately determined in consideration of various factors such as the gender, body weight, age, and symptoms of the subject. For example, the amount of the EVs is preferably 0.0001 to 100.0 mg, more preferably 0.001 to 10 mg, and particularly preferably 0.05 to 2.0 mg per 1 kg of the body weight of the subject.

The administration form is not particularly limited and is appropriately selected as necessary, but in general, the therapeutic agent can be administered as an oral preparation such as a tablet, a capsule, granules, fine granules, a powder, a liquid preparation, a syrup, a suspension, an emulsion, or an elixir, an injection, an infusion, a suppository, an inhalant, a mucoadhesive agent, or a spray.

The route of administration of the therapeutic agent according to the present embodiment may be, for example, inhalation, spray administration, injection, infusion, oral, transdermal, intranasal, topical, intravaginal, or rectal.

In addition, the number of times of administration may be three times a day, twice a day, once a day, every other day, every third day, once a week, once every two weeks, once a month, or the like. In addition, the administration period may be 1 day, 2 days, 3 days, 1 week, 2 weeks, 1 month, half a year, one year, or more.

The therapeutic agent according to the present embodiment preferably has at least one effect selected from the group consisting of an anti-fibrotic effect, an anti-aging effect, and a cell proliferation effect on epithelial cells, and more preferably has all the effects. In particular, the cell proliferation effect on epithelial cells is a new drug efficacy as compared with the conventional bronchodilator.

### [Composition for treating chronic respiratory diseases]

**In** one embodiment, the present invention provides a composition for treating chronic respiratory diseases, containing the above-described therapeutic agent for chronic respiratory diseases and an additive that is pharmaceutically acceptable.

The composition for treating chronic respiratory diseases according to the present embodiment can be administered orally in the form of, for example, a tablet, a capsule, granules, fine granules, a powder, a liquid preparation, a syrup, a suspension, an emulsion, or an elixir, or non-orally in the form of an injection, an infusion, a suppository, an inhalant, a mucoadhesive agent, a spray, or the like.

In the composition for treating chronic respiratory diseases according to the present embodiment, additives that are pharmaceutically acceptable and used in the formulation of a general pharmaceutical composition can be used without particular limitation. More specific examples thereof include excipients such as starch and crystalline cellulose; binding agents such as gelatin, corn starch, gum tragacanth, and gum arabic; swelling agents such as alginic acid; solvents for an injection such as water, ethanol, and glycerin; pressure sensitive adhesives such as rubber-based pressure sensitive adhesives and silicone-based pressure sensitive adhesives; lubricants such as calcium stearate and magnesium stearate; sweeteners such as sucrose, lactose, saccharin, and maltitol; flavoring agents such as peppermint and safflower oil; stabilizers such as benzyl alcohol and phenol; buffering agents such as phosphates and sodium acetate; solubilizing agents such as benzyl benzoate and benzyl alcohol; antioxidants; preservatives, and the like.

The additive can be used alone or in the form of a mixture of two or more types thereof.

The additives may be used alone or in the form of a mixture of two or more types thereof as a carrier of the therapeutic agent for chronic respiratory diseases.

### [Other Embodiments]

In one embodiment, the present invention provides EVs derived from a lipofibroblast for treating chronic respiratory diseases.

In one embodiment, the present invention provides a method for treating chronic respiratory diseases, the method including administering an effective amount of EVs derived from a lipofibroblast to a patient in need of treatment.

In one embodiment, the present invention provides the use of EVs derived from a lipofibroblast for manufacturing a therapeutic agent for chronic respiratory diseases or a composition for treating chronic respiratory diseases.

### [Quality evaluation kit]

In one embodiment, the present invention provides a quality evaluation kit for a therapeutic agent for chronic respiratory diseases, the kit containing an anti-LAT1 antibody. As described later in Examples, in a case where lipofibroblast-derived EVs are treated with a LAT1 inhibitor, the suppression of the induced expression of p21 and p16, which is one of the drug efficacies of the therapeutic agent for chronic respiratory diseases, is relieved. Therefore, it can be said that the expression level of LAT1 in lipofibroblast-derived EVs is related to the drug efficacy of the therapeutic agent for chronic respiratory diseases. Therefore, by evaluating the expression level of LAT1 in the lipofibroblast-derived EVs, it is possible to evaluate the quality of whether or not the therapeutic agent for chronic respiratory diseases has drug efficacy.

Furthermore, the kit may include an ELISA kit for detecting a LAT1 protein, in addition to the antibody.

### [Quality evaluation method]

In one embodiment, the present invention provides a quality evaluation method for a therapeutic agent for chronic respiratory diseases, the method using an anti-LAT1 antibody. In the quality evaluation method according to the present embodiment, the above-described quality evaluation kit may be used.

For example, the quality of the therapeutic agent for chronic respiratory diseases can be maintained by evaluating the EVs recovered from the culture supernatant of the lung fibroblast to which the metabolic regulating agent has been added, by the quality evaluation method according to the present embodiment.

### Examples

Hereinafter, the present invention will be described with reference to Examples, but the present invention is not limited to the following Examples.

### [Induction of lipofibroblast]

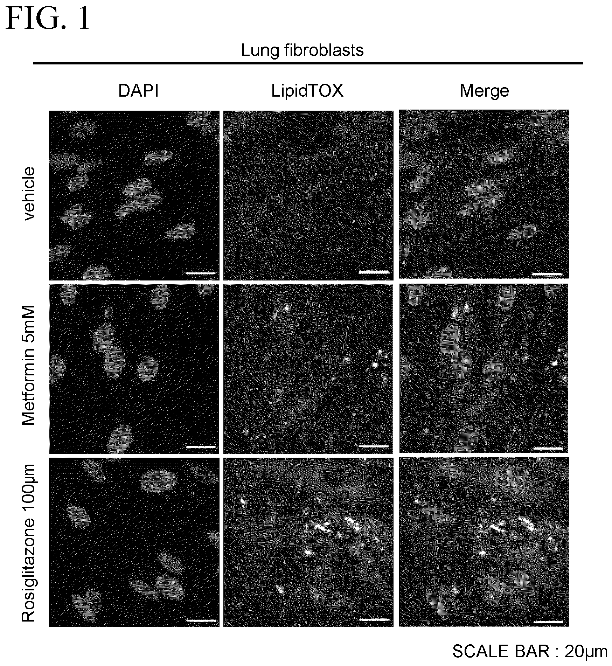

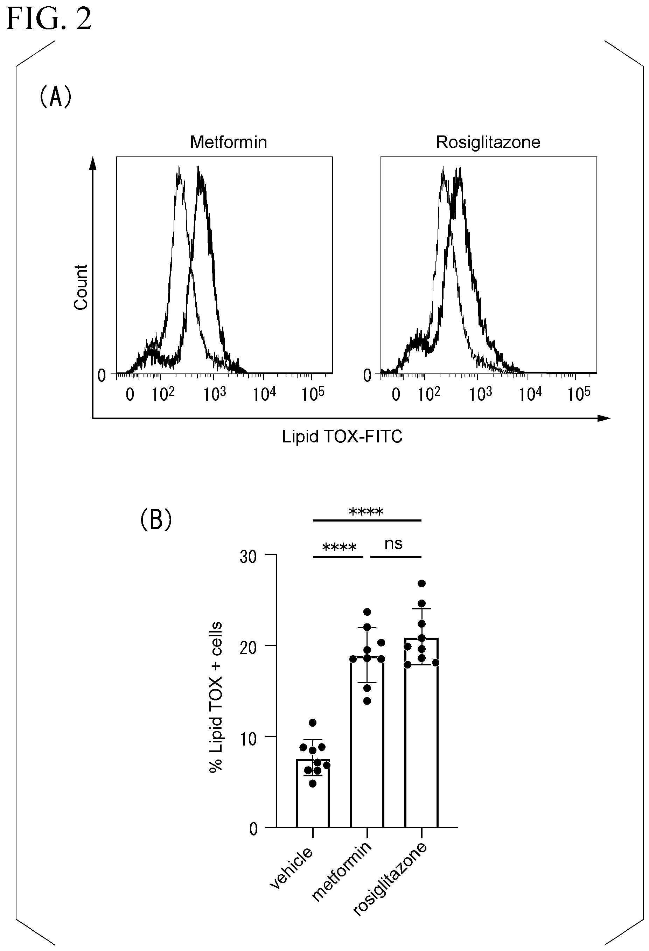

Metformin and Rosiglitazone were each added to the primary culture cells of the lung fibroblast derived from the healthy subject, and the cells were cultured for 72 hours. FIG. 1 shows the results of staining lipid droplets in the cytoplasm using LipidTOX. Lipid droplets were found in the cytoplasm of the cells to which Metformin and Rosiglitazone were each added. (A) of FIG. 2 shows the results of observing these cells using flow cytometry, and (B) of FIG. 2 shows the quantitative results thereof. It was confirmed that the LipidTOX-positive cells were significantly increased by the addition of an agent.

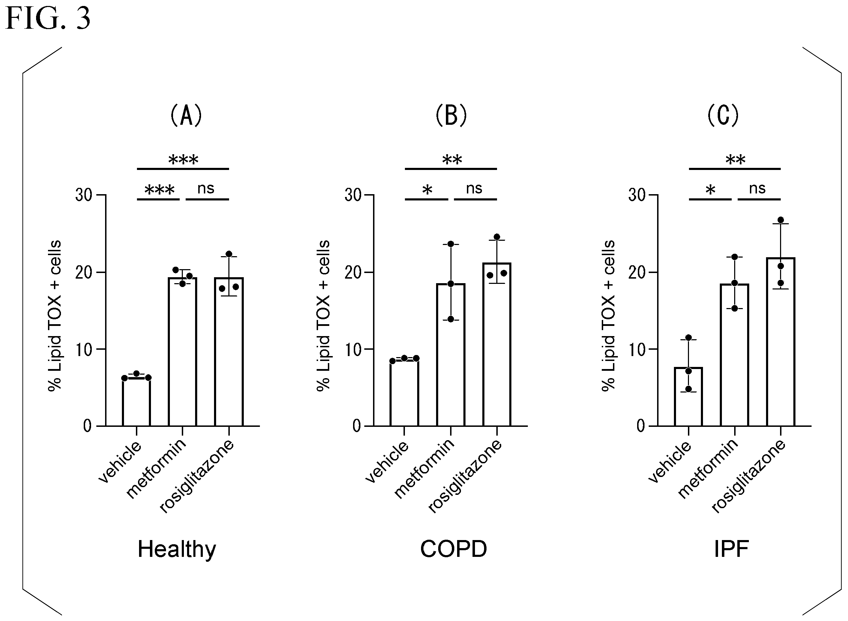

The same experiment was performed using primary culture cells of lung fibroblasts derived from a COPD patient and an idiopathic pulmonary fibrosis (IPF) patient. The results are shown in FIG. 3. It was confirmed that the lipofibroblasts were also induced in the lung fibroblasts derived from the COPD patient and the IPF patient, as in the lung fibroblasts derived from the healthy subject.

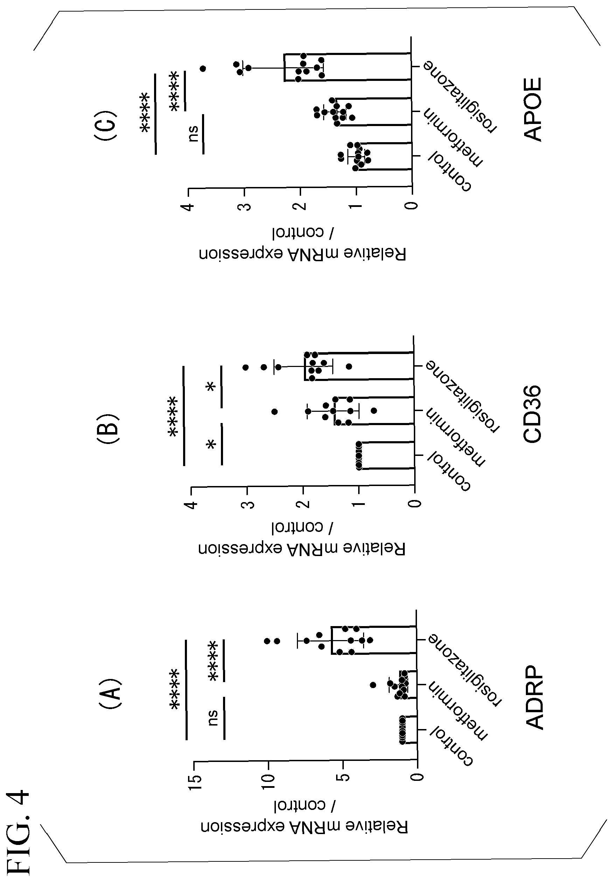

Expression of ADRP, which is a major marker of a lipofibroblast, and expression of CD36 and APOE, which are called lipogenic genes, were examined by using a culture obtained by adding each of Metformin and Rosiglitazone to primary culture cells of lung fibroblasts derived from a healthy subject and culturing for 72 hours. The results are shown in FIG. 4. In cells to which Metformin and Rosiglitazone were each added, an increase in the expression of these genes was confirmed.

### [Recovery of lipofibroblast-derived EVs]

Rosiglitazone was added to primary culture cells of lung fibroblasts derived from a healthy subject, and cells were cultured for 72 hours and then further cultured for 48 hours in the culture medium which had been replaced with Advanced DMEM. Thereafter, the culture supernatant was collected, centrifuged at 2,000 g at 4°C for 10 minutes, and filtered through a 0.22 µm filter. The filtered culture supernatant was subjected to ultracentrifugation at 210,000 g and 4°C for 45 minutes, the decantenation of the supernatant was performed, and washing with PBS was performed. Using this, the solution was further subjected to ultracentrifugation at 210,000 g and 4°C for 45 minutes, the decantenation of the supernatant was performed, and the remaining solution was collected as EVs.

### [Anti-fibrotic effect of lipofibroblast-derived EVs]

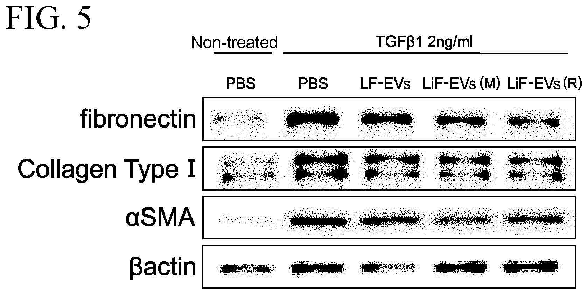

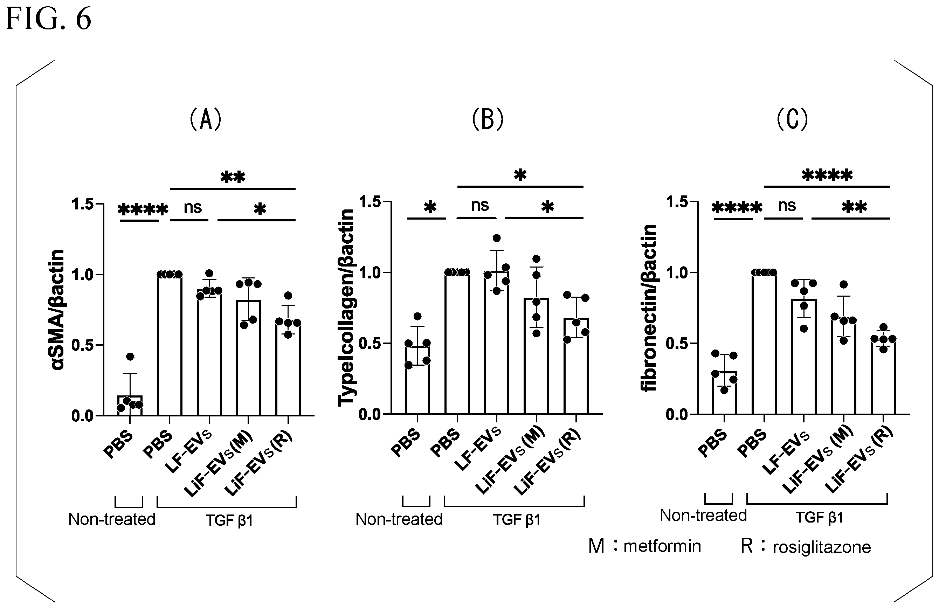

After adding 2 ng/mL of TGFβ1 and 10 µg/mL of EVs to primary culture cells of lung fibroblasts derived from healthy subjects, the expression of each protein was examined by Western blot (see FIG. 5). FIG. 6 shows the results of quantifying the signal intensity of FIG. 5. In lipofibroblast-derived EVs induced using Metformin or Rosiglitazone, the suppression of the induced expression of αSMA, type I collagen, and fibronectin was observed, and an anti-fibrotic effect against TGFβ was exhibited.

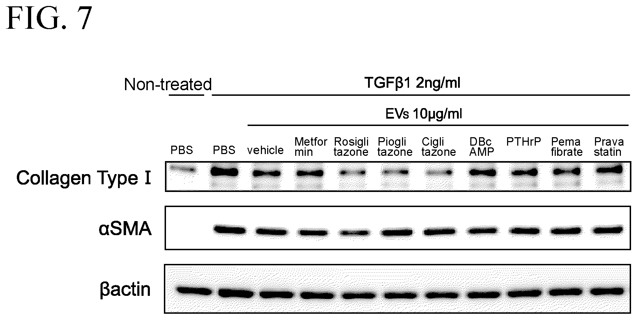

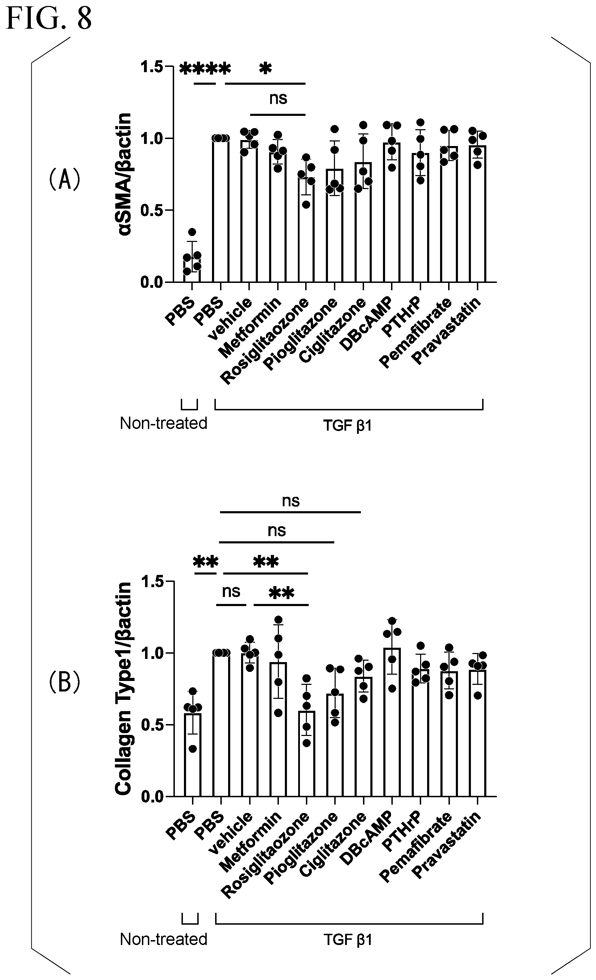

The same experiments were performed using various metabolically related agents. FIG. 7 shows the result of the Western blot, and FIG. 8 shows the result of quantifying the signal intensity. It was confirmed that the lipofibroblast-derived EVs induced by using Rosiglitazone exhibited the highest anti-fibrotic effect.

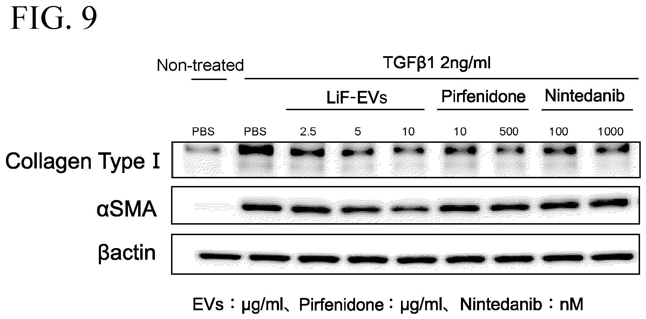

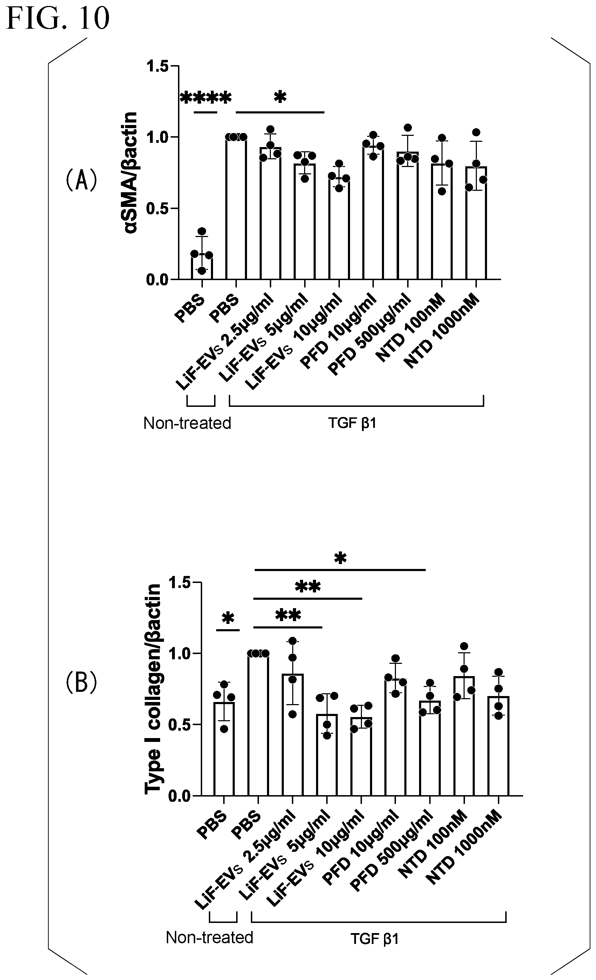

The same experiment was performed using each of Pirfenidon (PFD) and Nintedanib (NTD), which are existing antifibrotic agents. FIG. 9 shows the result of the Western blot, and FIG. 10 shows the result of quantifying the signal intensity. It was confirmed that the lipofibroblast-derived EVs exhibited an excellent effect as compared with Pirfenidon (PFD) and Nintedanib (NTD).

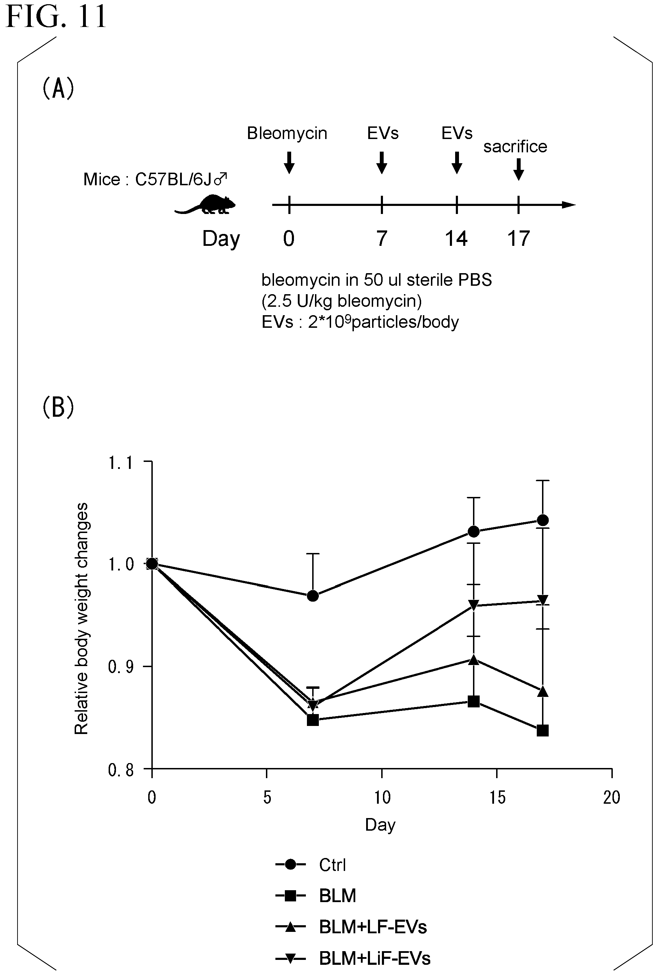

Bleomycin (BLM) was administered to mice to construct a pulmonary fibrosis model. Lung fibroblast-derived EVs (LF-EVs) or Rosiglitazone-induced lipofibroblast-derived EVs (LiF-EVs) were administered 7 days and 14 days after the administration of Bleomycin (see (A) of FIG. 11). (B) of FIG. 11 shows the results of measuring the body weight of each administration group. Although a decrease in body weight by the administration of Bleomycin was confirmed, a re-increase in body weight was observed in the group to which lipofibroblast-derived EVs were administered.

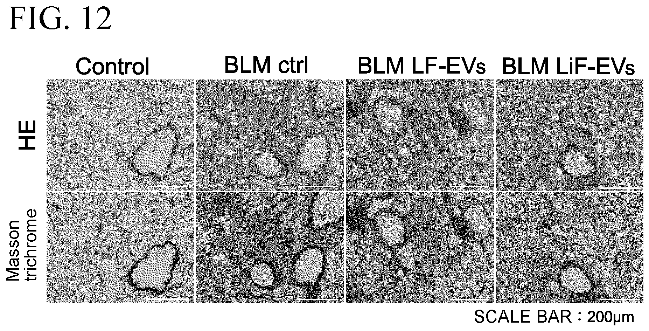

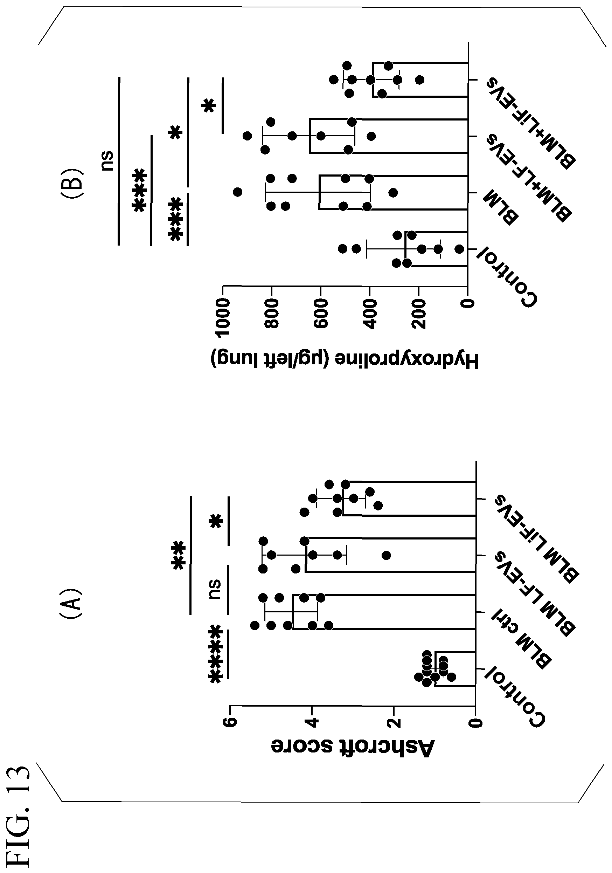

In addition, a pulmonary tissue section was produced from the mouse after the body weight measurement (see FIG. 12), Masson trichrome staining was performed, and the degree of severity of pulmonary fibrosis was evaluated by the Ashcroft scoring method (see (A) of FIG. 13). It was confirmed that the lipofibroblast-derived EVs induced using Rosiglitazone significantly suppressed fibrosis of a lung as compared with the lung fibroblast-derived EVs. The same results were also confirmed in the hydroxyproline assay using the homogenized lung samples (see FIG. 13 (B)).

### [Anti-aging effect of lipofibroblast-derived EVs]

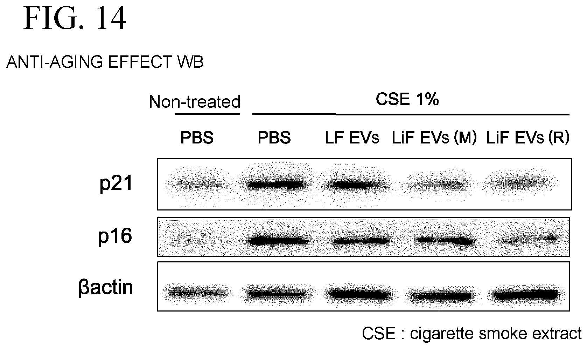

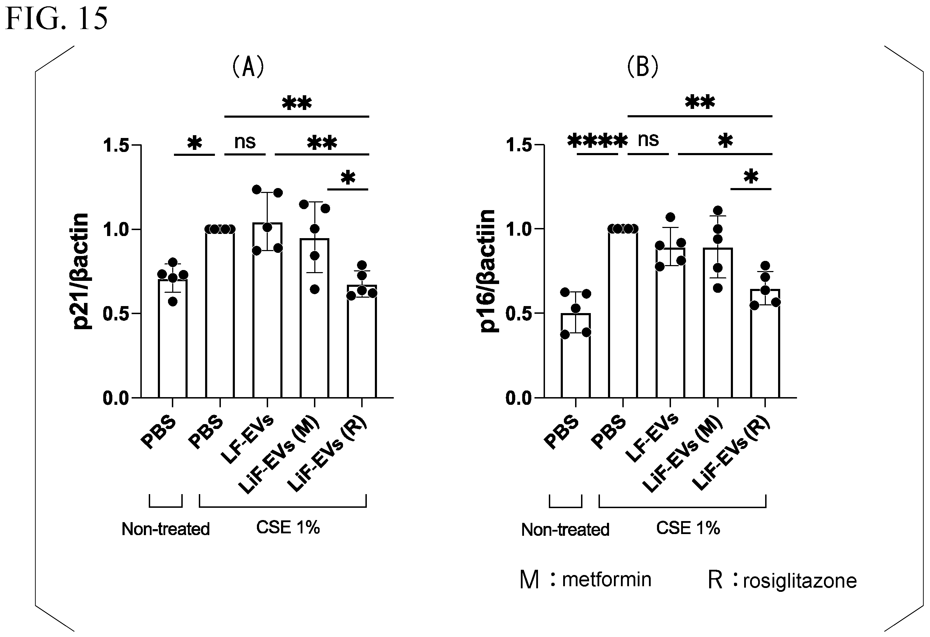

Human bronchial epithelial cells were cultured for 48 hours after adding 1% of cigarette smoke extract (CSE) and 10 µg/mL of EVs, and then the expression of each protein was examined by Western blot (see FIG. 14). In lipofibroblast-derived EVs induced using Rosiglitazone, the suppression of the induced expression of p21 and p16 was observed, and an anti-aging effect was exhibited (see (A) and (B) of FIG. 15).

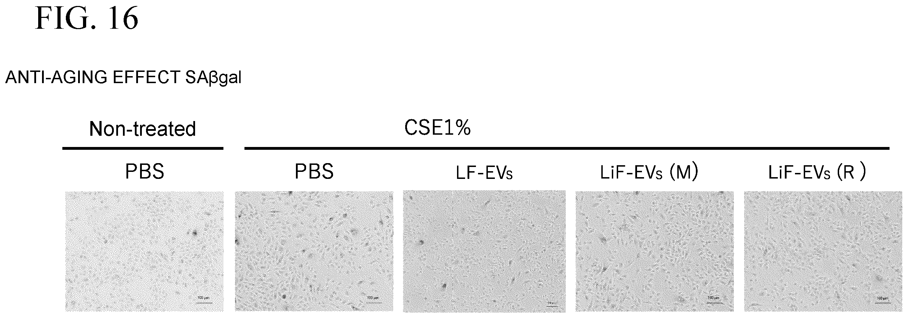

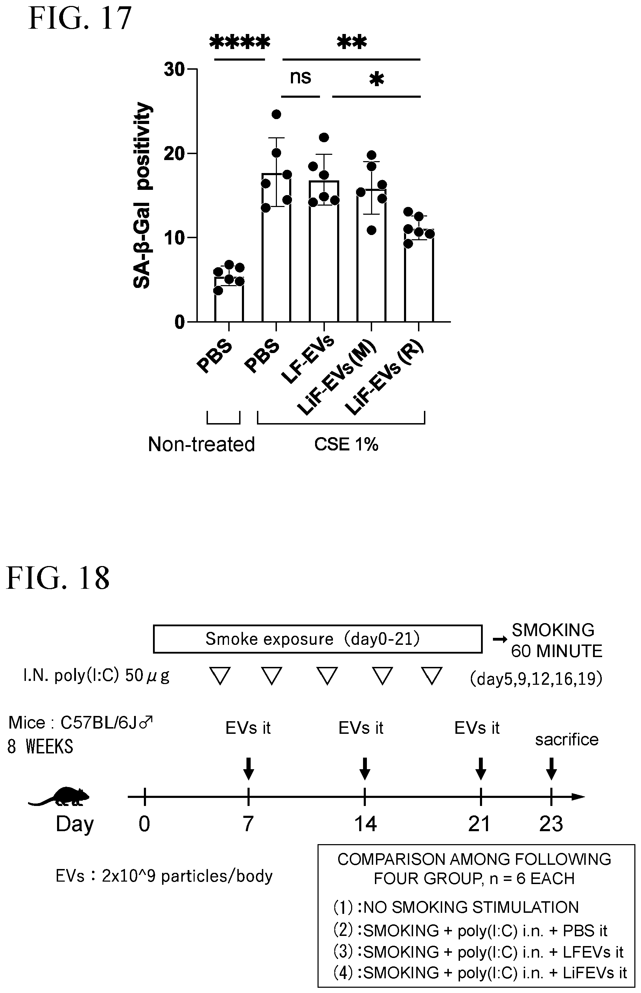

In addition, SA-β-gal staining was performed on the cells after the treatment (see FIG. 16). FIG. 17 shows the proportion of SA-β-gal positive cells. It was confirmed that the lipofibroblast-derived EVs induced using Rosiglitazone also exhibited the highest anti-aging effect in the SA-β-gal staining.

### [Effect of lipofibroblast-derived EVs on short-term smoking-exposed model mouse]

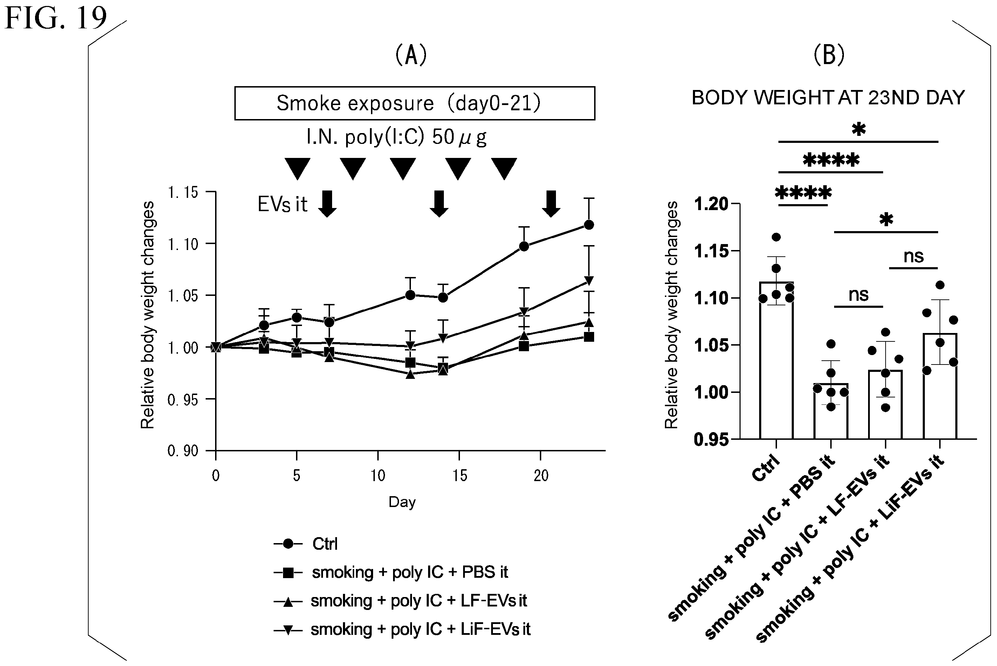

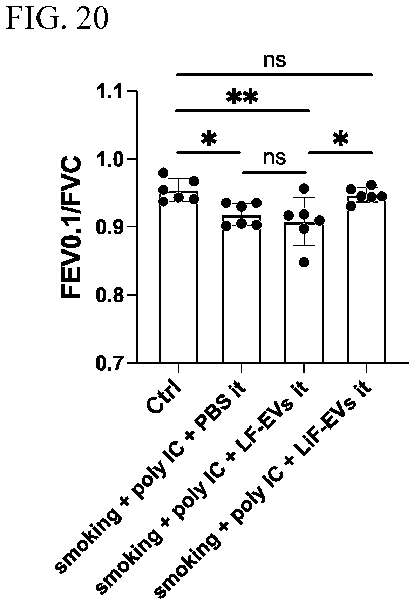

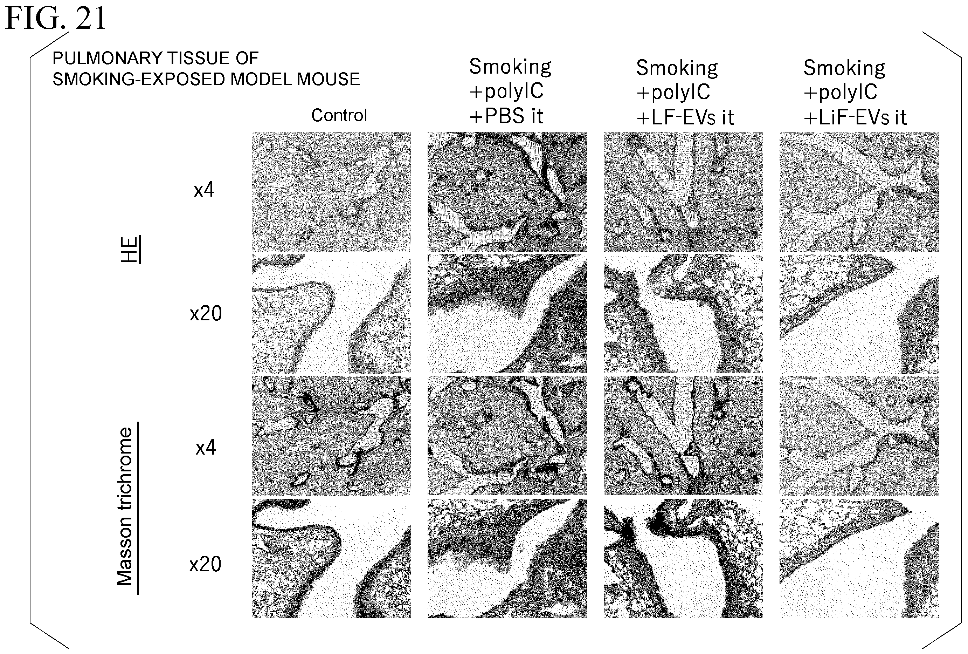

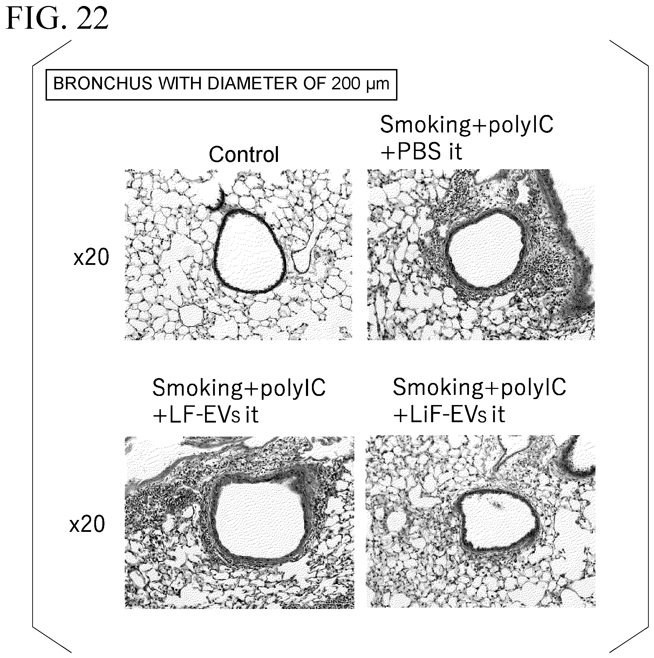

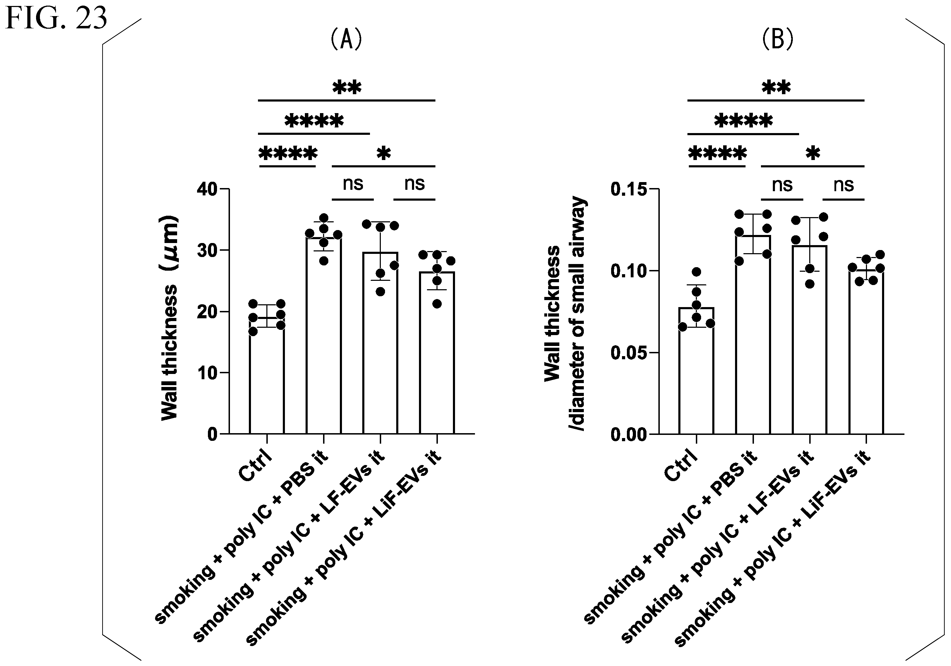

In the administration schedule shown in FIG. 18, lipofibroblast-derived EVs were administered to smoking-exposed model mouse (short-term exposure model). The results are shown in (A) and (B) of FIG. 19. In the lipofibroblast-derived EVs administration group, the suppression of decrease in the body weight was observed. In addition, a respiratory function test was performed using a flexiVent with these mice. As shown in FIG. 20, it was confirmed that FEV0.1/FVC was decreased by the smoking stimulation and the deterioration of the respiratory function was suppressed in the administration of the lipofibroblast-derived EVs. In addition, a pulmonary tissue section was produced from the similarly treated mouse, and Masson trichrome staining was performed (see FIG. 21). In the control, it was confirmed that inflammatory cell infiltration occurred in the bronchus and the perivascular region because of smoking and stimulation with poly(I:C). On the other hand, in the lipofibroblast-derived EVs administration group, the suppression of inflammatory cell infiltration was confirmed. Furthermore, the wall thickness of the bronchus having a diameter of about 200 µm was observed (see FIG. 22). FIG. 23 shows the result of quantifying the wall thickness of the bronchus, or the wall thickness of the bronchus/the diameter of the small airway. In the control, thickening of the bronchial wall and inflammatory cell infiltration in the periphery were observed because of smoking and poly (I:C) stimulation, but it was confirmed that these were suppressed in the lipofibroblast-derived EVs administration group.

### [Epithelial cell proliferation effect of lipofibroblast-derived EVs]

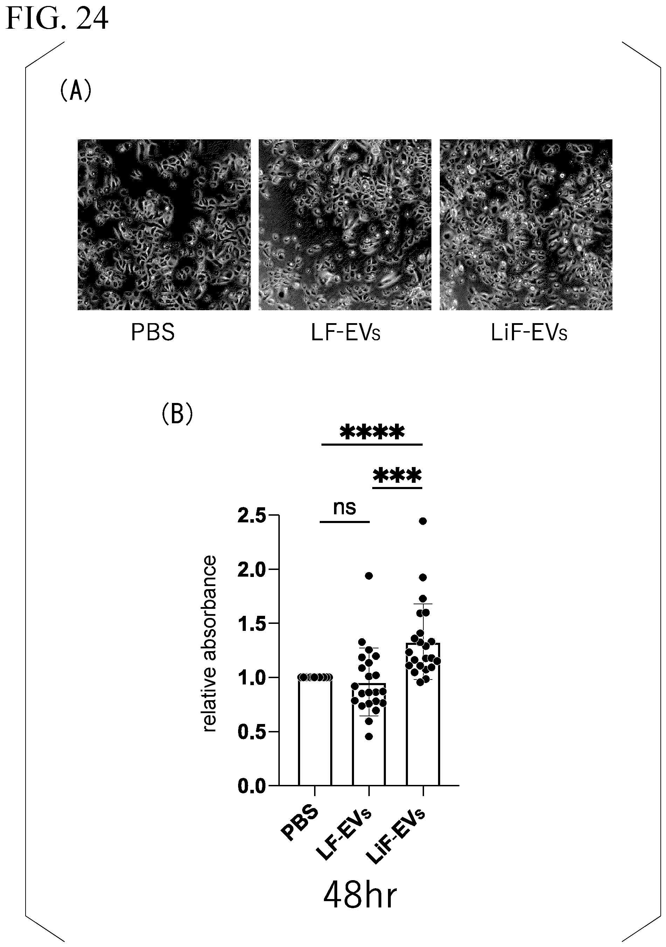

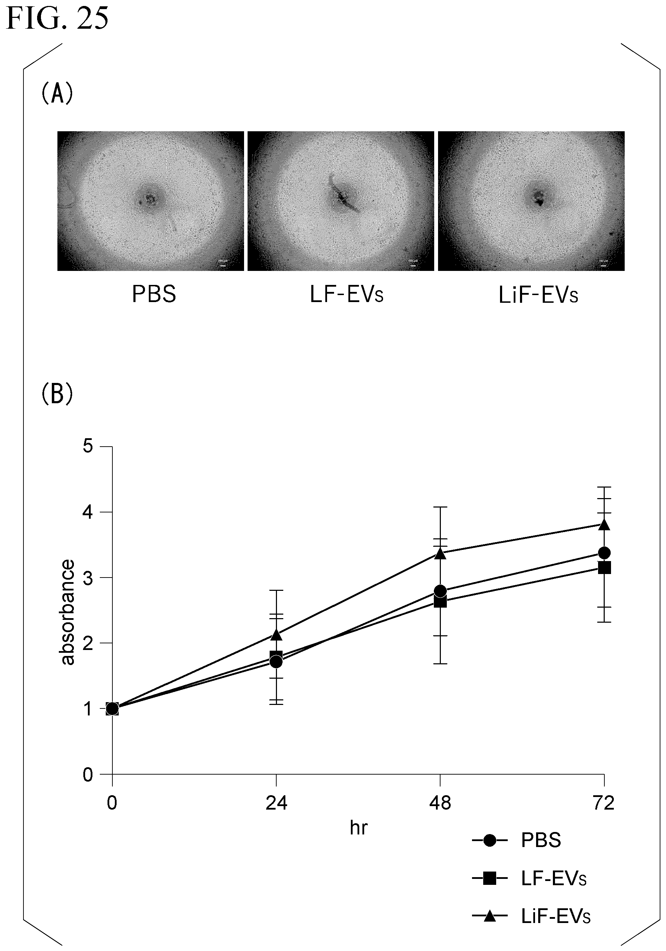

HBEC was subjected to planar culture with addition of lipofibroblast-derived EVs. (A) of FIG. 24 shows a microscopic observation image of cells, and (B) of FIG. 24 shows the results of the CCK-8 assay. Similarly, HSAEC was subjected to planar culture with addition of lipofibroblast-derived EVs. (A) of FIG. 25 shows a microscopic observation image of cells, and (B) of FIG. 25 shows the results of the CCK-8 assay. In both HBEC and HSAEC, it was confirmed that the addition of lipofibroblast-derived EVs promoted cell proliferation.

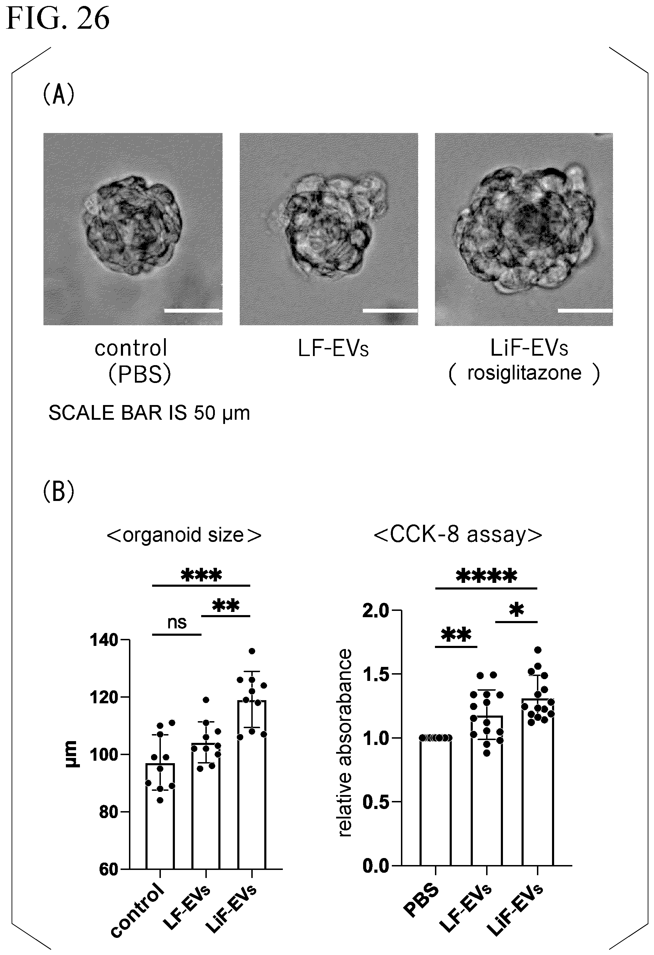

In addition, HBEC was subjected to 3D culture using Matrigel. (A) of FIG. 26 shows a microscopic observation image of cells, and (B) of FIG. 26 shows the results of organoid size and the CCK-8 assay. It was confirmed that the addition of lipofibroblast-derived EVs promoted cell proliferation and increased the organoid size.

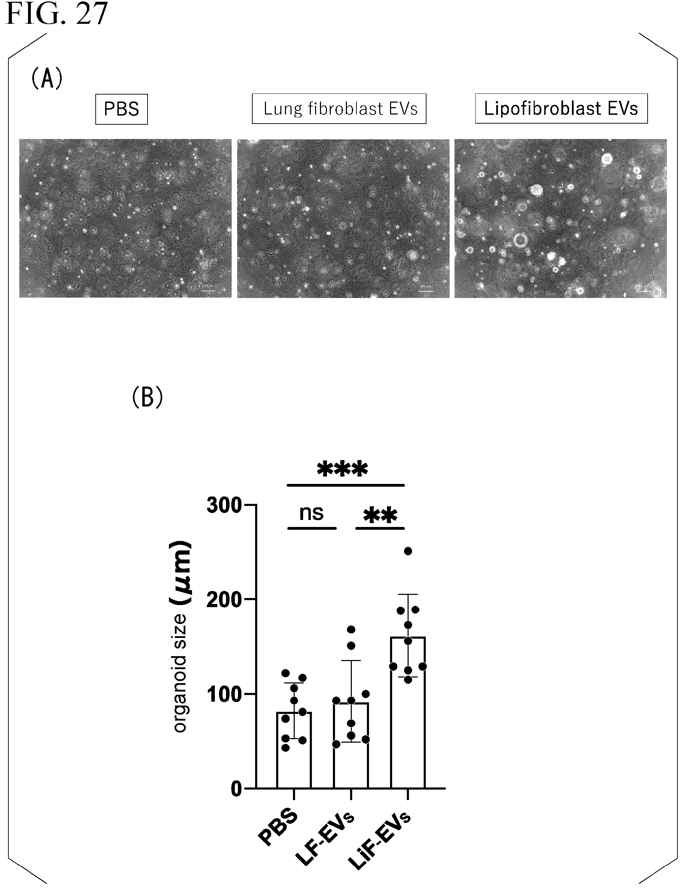

Furthermore, alveolar type II cells (AT2) were separated from the surgical lung specimen using a cell sorter, and AT2 were subjected to 3D culture using Matrigel. (A) of FIG. 27 shows a microscopic observation image of cells, and (B) of FIG. 27 shows the results of the organoid size and the CCK-8 assay. It was confirmed that also in AT2, the addition of lipofibroblast-derived EVs promoted cell proliferation and increased the organoid size.

### [Effect of lipofibroblast-derived EVs on long-term smoking-exposed model mouse]

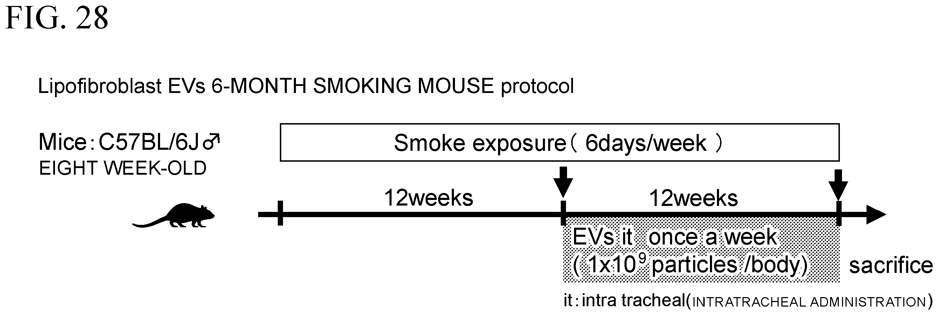

In the administration schedule shown in FIG. 28, lipofibroblast-derived EVs were administered to smoking-exposed model mouse (long-term exposure model). Specifically, the experiment was performed with four separate group: a control group (ctrl) to which no smoking exposure stimulation was subjected; a group (CS + PBS) to which smoking exposure for 6 months and PBS it (intratracheal administration) were subjected; a group (CS + LF EVs) to which smoking exposure for 6 months and LF EVs it were subjected; and a group (CS + LiF EVs) to which smoking exposure for 6 months and LiF EVs it were subjected.

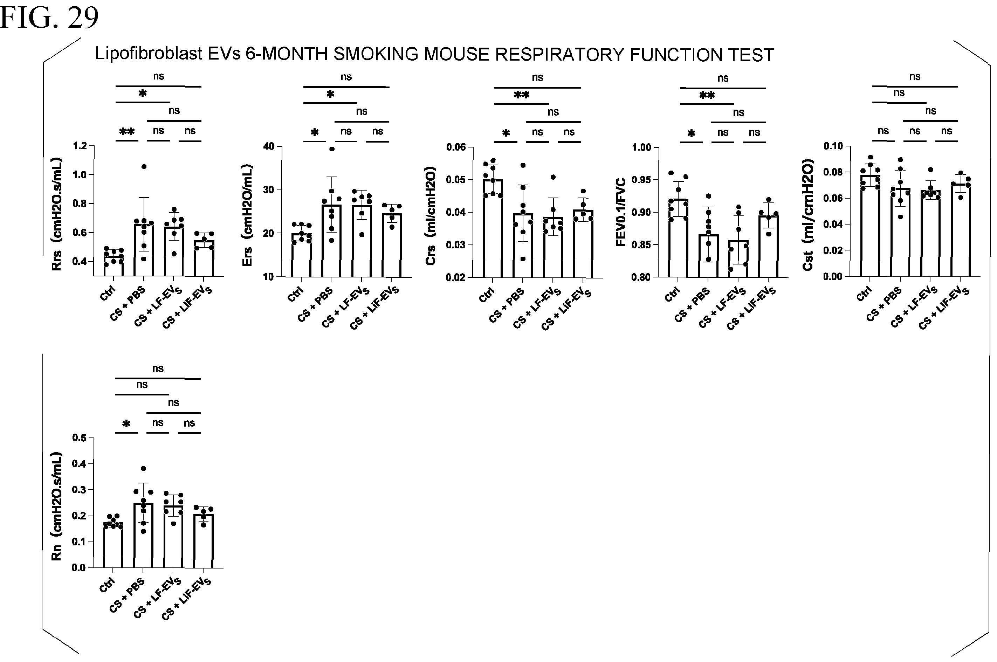

A respiratory function test was performed using a flexiVent with these mice. The results are shown in FIG. 29. In the PBS group, an increase in airway resistance, a decrease in lung compliance (ease of lung inflation), and a decrease in FEV0.1/FVC (0.1 second rate) were observed, and the respiratory function was deteriorated. On the other hand, as shown in FIG. 29, it was confirmed that the decrease in respiratory function was suppressed in the group in which the lipofibroblast-derived EVs were administered via the airway.

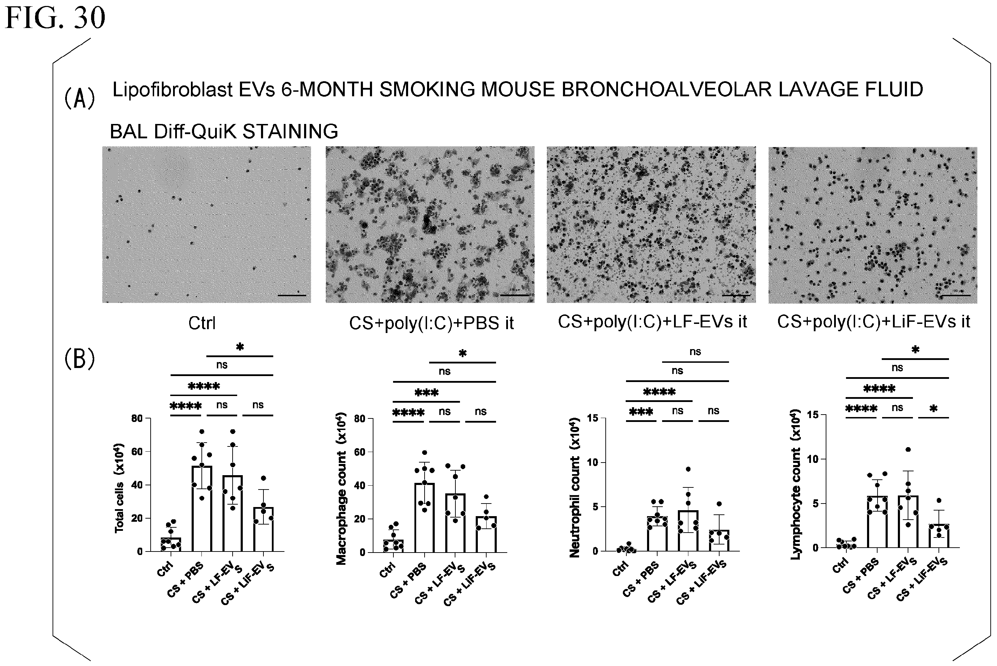

In addition, the evaluation of the inflammatory cells in the bronchoalveolar lavage (BAL) fluid in the 6-month smoking mouse was performed. (A) of FIG. 30 shows Diff-Quik-stained images, and (B) of FIG. 30 shows the total number of cells, the number of macrophages, the number of neutrophils, and the number of lymphocytes. Inflammatory cells in the bronchoalveolar lavage fluid increased because of the smoking stimulation, but this increase was suppressed in the lipofibroblast-derived EVs administration group. As shown in (B) of FIG. 30, in the lipofibroblast-derived EVs administration group, the increase in the total number of cells, the number of macrophages, the number of neutrophils, and the number of lymphocytes was suppressed.

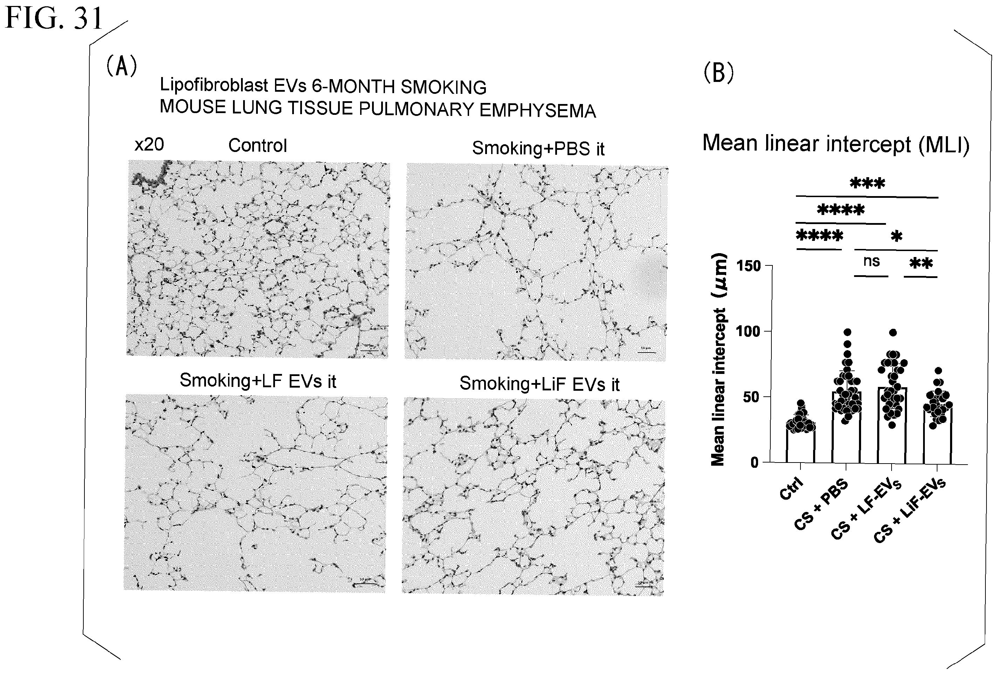

In addition, the evaluation of the pulmonary emphysema in the pulmonary tissue of the 6-month smoking mouse was performed. (A) of FIG. 31 shows an HE-stained image, and (B) of FIG. 30 shows a quantitative result of the mean linear intercept (MLI). Although pulmonary emphysema was formed by smoking stimulation, the formation of pulmonary emphysema was suppressed in the lipofibroblast-derived EVs administration group.

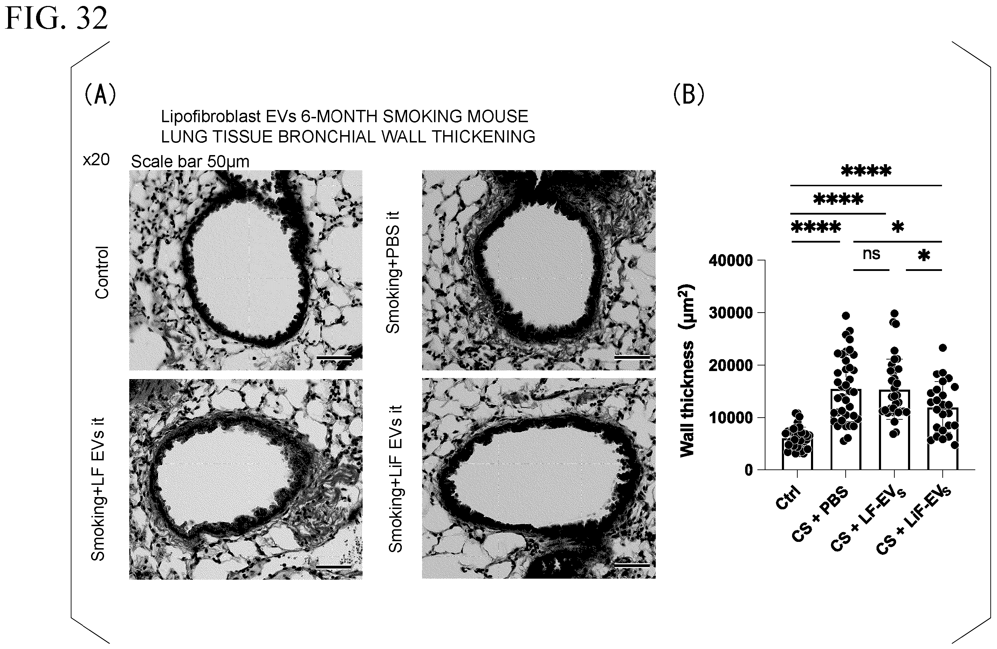

In addition, the bronchial wall thickening in the pulmonary tissue of the 6-month smoking mouse was performed. (A) of FIG. 32 shows a Masson trichrome-stained image, and (B) of FIG. 32 shows the quantitative result of the thickness of the bronchial wall. Although fibrosis occurs around the trachea because of smoking stimulation, and the bronchial wall is thickened, the thickening is suppressed in the lipofibroblast-derived EVs administration group.

As described above, in the 6-month smoking COPD mouse model, the decrease in respiratory function, the inflammatory cell infiltration in the bronchoalveolar lavage fluid, the pulmonary emphysema formation, and the bronchial wall thickening, all due to smoking stimulation, were suppressed by the lipofibroblast-derived EVs.

### [Analysis of protein in lipofibroblast-derived EVs]

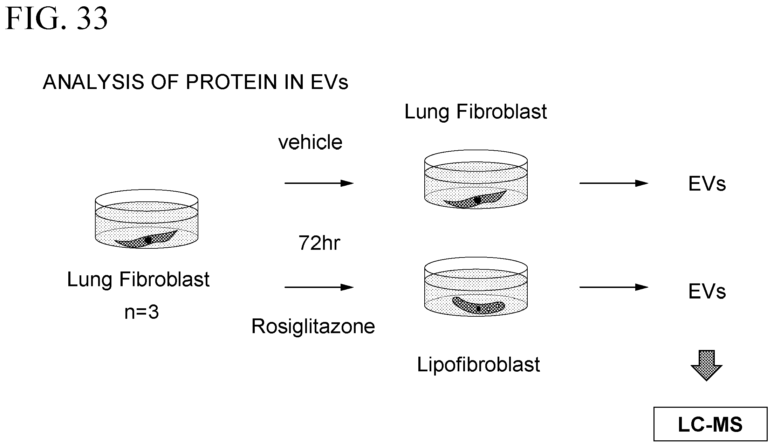

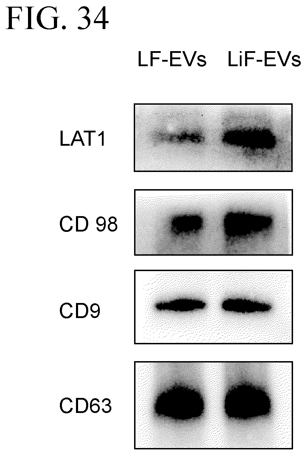

Three samples of EVs of lung fibroblasts and three samples of EVs of lipofibroblasts were collected and analyzed by LC-MS (mass spectrometry) (see FIG. 33). In a case where proteins having a significant difference and a fold change > 1.5 (a difference of 1.5 times or more) were extracted, 45 types of proteins were identified. Among these proteins, LAT1 and CD98 were focused on. It is known that CD98 forms a complex with LAT1 and functions. LAT1 is a 12-transmembrane type membrane protein and is an amino acid transporter that transports large neutral amino acids such as leucine, isoleucine, and valine. FIG. 34 is a result of Western blot analysis performed on lung fibroblast-derived EVs and lipofibroblast-derived EVs. It was confirmed that the expression of both LAT1 and CD98 was increased in the EV of the lipofibroblast. The increase in the expression of both molecules suggests that the function of LAT1 is maintained.

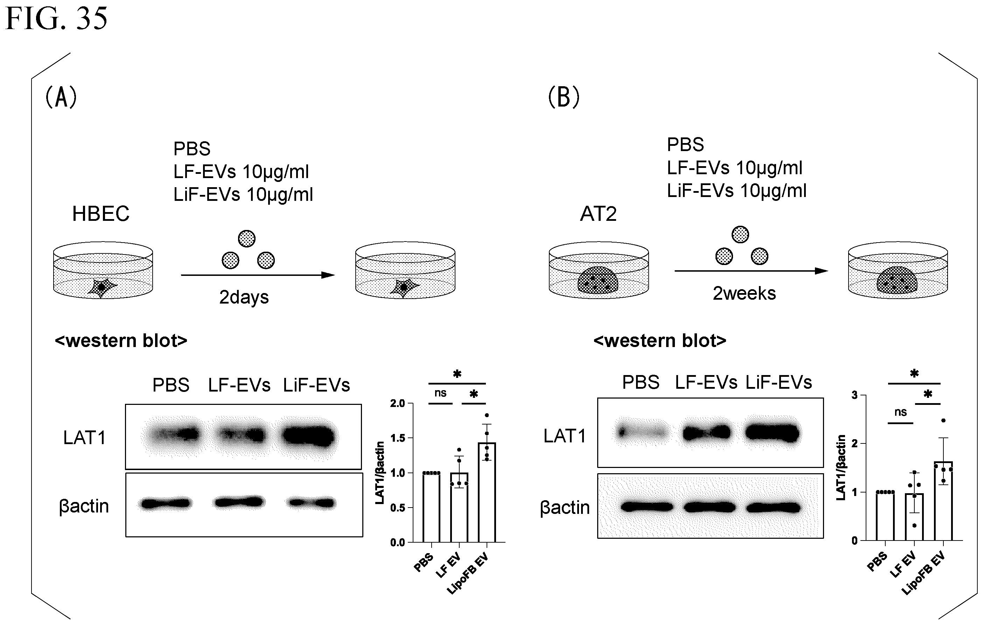

The change in the expression level of LAT1 with the addition of lipofibroblast-derived EVs to each of HBEC cells and AT2 cells was confirmed. FIG. 35 shows the results. It was confirmed that the expression of LAT1 with the addition of lipofibroblast-derived EVs was increased in both cells.

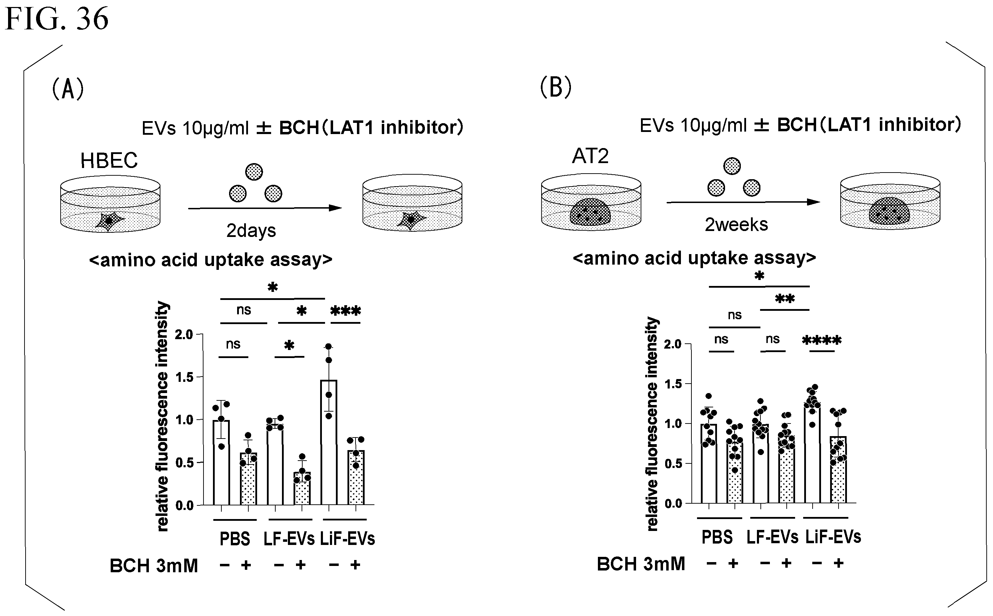

The change in the uptake of amino acids in cells by LAT1 was evaluated by an amino acid uptake assay. FIG. 36 shows the results. It was confirmed that in a case where lipofibroblast-derived EVs were added to each of HBEC cells and AT2 cells, the uptake of amino acids by cells was increased. On the other hand, it was confirmed that the administration of BCH, which is a LAT1 inhibitor, resulted in no increase in the uptake of amino acids. From the above, it was confirmed that the lipofibroblast-derived EVs increased the uptake of amino acids by cells via LAT1.

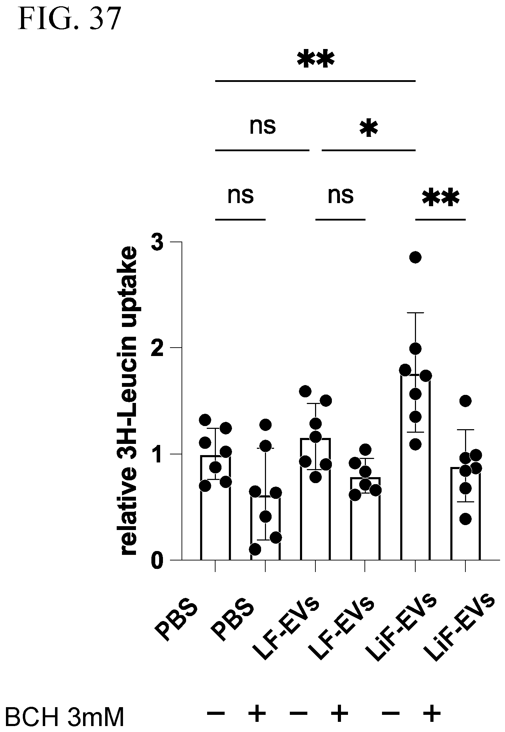

In HBEC cells, the change in the uptake of amino acids by LAT1 was evaluated by a ³H-L-Leu uptake experiment. FIG. 37 shows the results. It was confirmed that the addition of lipofibroblast-derived EVs increases the uptake of ³H-L-Leu. On the other hand, it was confirmed that the uptake of leucin was inhibited by BCH.

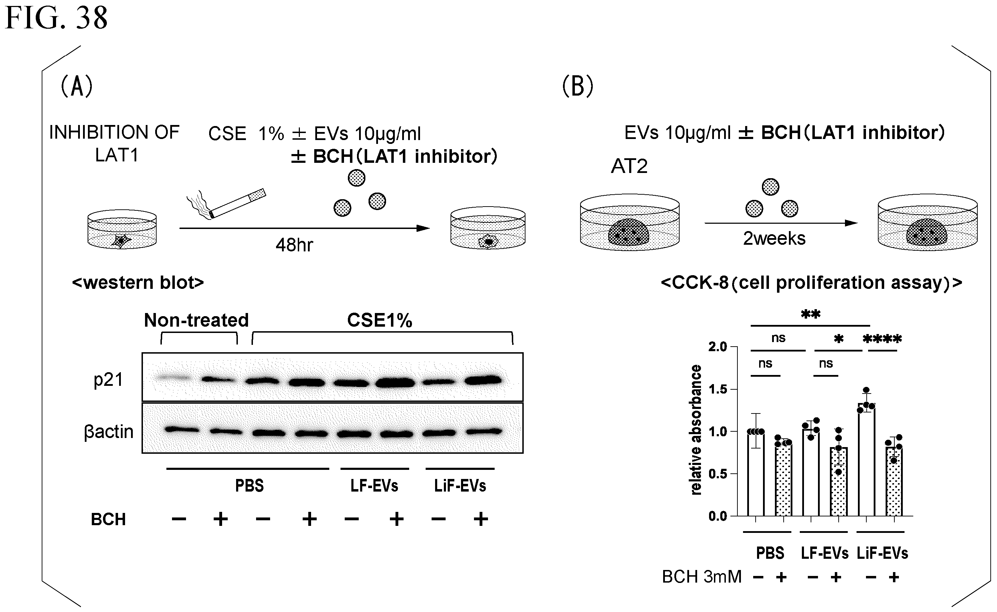

The expression of each protein was examined by Western blot after adding 1% of cigarette smoke extract (CSE), 10 µg/mL of EVs, and BCH to HBEC cells and culturing for 48 hours (see (A) of FIG. 38). It was confirmed that the suppression of the induced expression of p21 and p16 observed in the lipofibroblast-derived EVs was relieved by BCH.

In addition, AT2 was subjected to 3D culture using Matrigel with the addition of the lipofibroblast-derived EVs and BCH. (B) of FIG. 38 shows the results of the CCK-8 assay. It was confirmed that with the addition of lipofibroblast-derived EVs, the promotion of cell proliferation was relieved by BCH.

As described above, it has been confirmed that the effect of suppressing the aging of airway epithelial cells due to smoking stimulation and the effect of promoting the cell proliferation of AT2, which are the effects of the lipofibroblast-derived EVs, are relieved by the addition of the LAT1 inhibitor (BCH).

### INDUSTRIAL APPLICABILITY

According to the present invention, the present invention contributes to the treatment of chronic respiratory diseases.

## Claims

1. A therapeutic agent for chronic respiratory diseases, comprising:
extracellular vesicles from a lipofibroblast as an active component.

2. The therapeutic agent for chronic respiratory diseases according to Claim 1, wherein the extracellular vesicles from a lipofibroblast express L-type amino acid transporter 1 (LAT1).

3. The therapeutic agent for chronic respiratory diseases according to Claim 1, wherein the lipofibroblast is differentiated from a lung fibroblast by a metabolic regulating agent.

4. The therapeutic agent for chronic respiratory diseases according to Claim 3, wherein the metabolic regulating agent is at least one selected from the group consisting of Metformin, Rosiglitazone, Pioglitazone, Ciglitazone, Dibutyryl cyclic AMP (DBcAMP), Parathyroid hormone-related peptide (PTHrP), Pemafibrate, and Pravastatin.

5. The therapeutic agent for chronic respiratory diseases according to Claim 3, wherein the metabolic regulating agent is Rosiglitazone.

6. The therapeutic agent for chronic respiratory diseases according to Claim 1, wherein the therapeutic agent has an anti-fibrotic effect.

7. The therapeutic agent for chronic respiratory diseases according to Claim 1, wherein the therapeutic agent has an anti-aging effect.

8. The therapeutic agent for chronic respiratory diseases according to Claim 1, wherein the therapeutic agent has a cell proliferation effect on epithelial cells.

9. A composition for treating chronic respiratory diseases, comprising:
the therapeutic agent for chronic respiratory diseases according to any one of Claims 1 to 8; and
a pharmaceutically acceptable additive.

10. A quality evaluation kit for the therapeutic agent for chronic respiratory diseases according to any one of Claims 1 to 8, the quality evaluation kit comprising:
an anti-LAT1 antibody.

11. A quality evaluation method for the therapeutic agent for chronic respiratory diseases according to any one of Claims 1 to 8, the quality evaluation method comprising:
using an anti-LAT1 antibody.
